(19) Europäisches Patentamt
European Patent Office
Office européen des brevets

(11) Veröffentlichungsnummer: **0 173 319**
**A1**

(12) **EUROPÄISCHE PATENTANMELDUNG**

(21) Anmeldenummer: **85110831.6**

(22) Anmeldetag: **19.08.85**

(51) Int. Cl.⁴: **C 07 D 239/42**, A 01 N 47/44

(30) Priorität: **30.08.84 DE 3431918**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

(54) **Substituierte Arylsulfonylguanidine.**

(57) Die Erfindung betrifft neue substituierte Arylsulfonylguanidine der allgemeinen Formel (I)

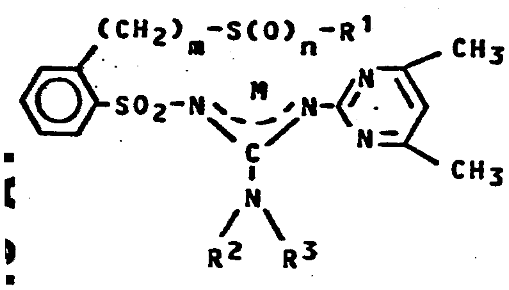

(I)

(worin die Reste M, $R^1$, $R^2$, $R^3$ und die Indizes m und n, die in der Beschreibung angegebene Bedeutung haben), sowie 1:1-Addukte von Verbindungen der Formel (I) mit starken Säuren, Verfahren zu ihrer Herstellung und ihre Verwendung als Herbizide.

0 173 319

BAYER AKTIENGESELLSCHAFT 5090 Leverkusen, Bayerwerk
Konzernverwaltung RP Bi/sam.
Patentabteilung Ib

# Substituierte Arylsulfonylguanidine

Die Erfindung betrifft neue substituierte Arylsulfonylguanidine, Verfahren zu ihrer Herstellung und ihre Verwendung als Herbizide.

Verschiedene Guanidin-Derivate sind aus Patentschriften (vgl. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt. Weitere Guanidin-Derivate sind Gegenstand einer nicht zum vorveröffentlichten Stand der Technik gehörenden Patentanmeldung der Anmelderin (vgl. DE-OS 33 34 455).

Es wurden nun neue substituierte Arylsulfonylguanidine der allgemeinen Formel (I)

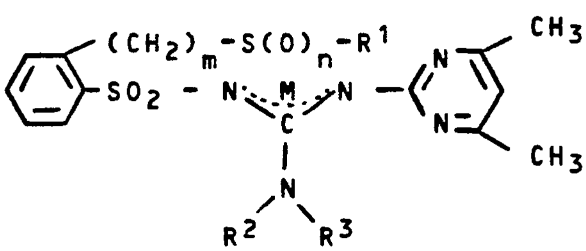

(I)

in welcher

m für Null oder 1 steht,

n für Null, 1 oder 2 steht,

M für Wasserstoff oder ein Metalläquivalent steht,

$R^1$ für $C_1$-$C_6$-Alkyl [welches gegebenenfalls durch Fluor und/oder Chlor substituiert ist], Amino, $C_1$-$C_4$-Alkylamino, $C_1$-$C_8$-Alkoxyamino, Di($C_1$-$C_4$-alkyl)-amino, $C_1$-$C_4$-Alkoxy-$C_1$-$C_4$-alkylamino, Benzyloxyamino oder Dimethylhydrazino steht,

$R^2$ für Wasserstoff, $C_1$-$C_6$-Alkyl [welches gegebenenfalls durch Fluor, Chlor, Brom, Cyano, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für den Rest

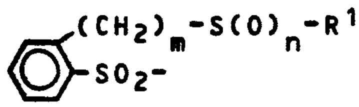 steht,

worin $R^1$ die oben angegebene Bedeutung hat,
m für Null oder 1 steht und
n für Null, 1 oder 2 steht;
in welcher weiter

$R^3$ für Wasserstoff, $C_1$-$C_6$-Alkyl (welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-C6-Alkinyl, Phenylethyl oder Benzyl [welches gegebenenfalls durch Fluor, Chlor, Nitro, Cyano, $C_1$-$C_4$-Alkyl,

$C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht,oder

$R^2$ und $R^3$ gemeinsam für $C_4$-$C_6$-Alkandiyl stehen, welches gegebenenfalls durch eine Sauerstoff-Brücke oder durch eine Brücke >N-$R^4$ unterbrochen ist, wobei

$R^4$ für $C_1$-$C_4$-Alkyl, $C_1$-$C_4$-Alkyl-carbonyl oder Phenyl steht [welches gegebenenfalls durch Fluor, Chlor, Brom, Cyano, Nitro, $C_1$-$C_4$-Alkyl, Trifluormethyl oder $C_1$-$C_4$-Alkoxy substituiert ist];

in welcher weiter

$R^3$ für den Rest -O-$R^5$ steht, worin

$R^5$ für $C_1$-$C_{12}$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], $C_3$-$C_6$-Alkenyl, $C_1$-$C_4$-Alkoxy-carbonyl-$C_1$-$C_2$-alkyl, Aminocarbonylmethyl, $C_1$-$C_4$-Alkylamino-carbonyl-methyl, Di-($C_1$-$C_4$-alkyl)-amino-carbonyl-methyl, Phenyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert ist] steht;

in welcher weiter

$R^3$ für den Rest $-N<^{R^6}_{R^7}$ steht, worin

$R^6$ für Wasserstoff oder $C_1$-$C_4$-Alkyl steht und

$R^7$ für Wasserstoff, $C_1$-$C_4$-Alkyl (welches gegebenenfalls durch

0 173 319

Fluor, Chlor, Brom, Cyano, $C_1$-$C_4$-Alkoxy oder $C_1$-$C_4$-Alkoxycarbonyl substituiert ist), $C_3$-$C_6$-Cycloalkyl, Phenylethyl, Benzyl oder Phenyl [welches gegebenenfalls durch Fluor, Chlor, Brom, Nitro, Cyano, $C_1$-$C_4$-Alkyl, $C_1$-$C_4$-Alkoxy oder $C_1$-$C_4$-Alkoxy-carbonyl substituiert ist] steht,

sowie 1:1-Addukte von Verbindungen der Formel (I) mit starken Säuren gefunden.

Die allgemeine Formel (I) steht - wenn M für Wasserstoff steht - für die einzelnen Tautomeren der Formel (IA) und (IB)

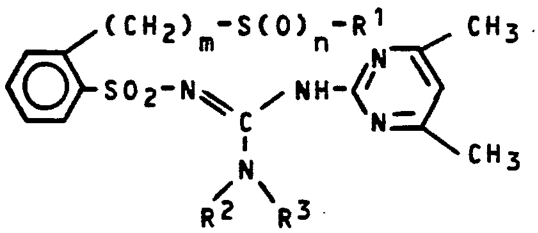

(IA)

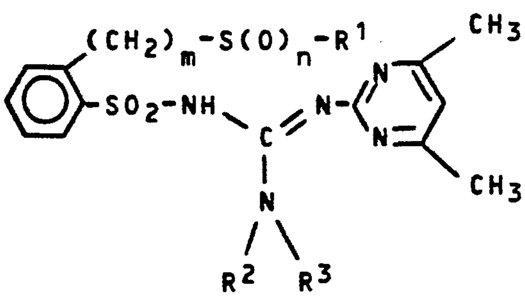

(IB)

in welchen

$R^1$, $R^2$, $R^3$, m und n die oben angegebenen Bedeutungen haben,

sowie für Gemische der Tautomeren (IA) und (IB).

Das Mischungsverhältnis (IA), (IB) hängt von aggregationsbestimmenden Faktoren, wie z.B. Temperatur, Lösungsmittel und Konzentration ab.

Für den Fall, daß neben M auch $R^2$ für Wasserstoff steht, ist eine weitere tautomere Form (IC) möglich:

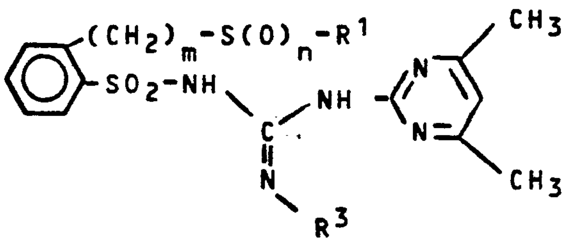

(IC)

Man erhält die neuen substituierten Arylsulfonylguanidine der Formel (I)

(a) für den Fall, daß M für Wasserstoff steht und

$R^2$ für den Rest 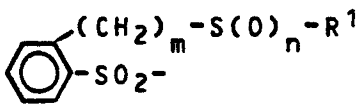 steht,

wenn man Guanidin-Derivate der Formel (II)

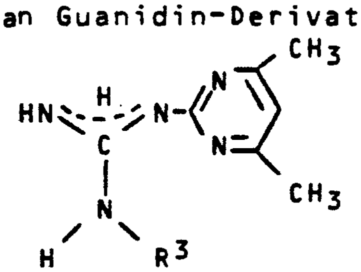

(II)

in welcher

$R^3$ die oben angegebene Bedeutung hat,

mit wenigstens zwei Moläquivalenten von substituierten Arensulfonsäurechloriden der Formel (III)

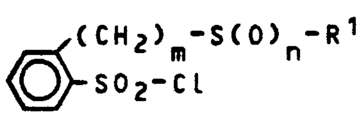

(III)

in welcher

$R^1$ sowie M und n die oben angegebenen Bedeutungen haben,

in Gegenwart von Säureakzeptoren und gegebenenfalls in Gegenwart von Verdünnungsmitteln umsetzt;
oder

(b) für den Fall, daß M für Wasserstoff steht und $R^2$ für Wasserstoff, $C_1$-$C_6$-Alkyl [welches gegebenenfalls wie oben angegeben substituiert ist], $C_3$-$C_6$-Cycloalkyl, $C_3$-$C_6$-Alkenyl oder Benzyl [ welches gegebenenfalls wie oben angegeben substituiert ist] steht oder zusammen mit $R^3$ die oben angegebene Bedeutung hat,

wenn man die nach dem unter (a) angegebenen Verfahren erhältlichen substituierten Arylsulfonylguanidine der Formel (ID)

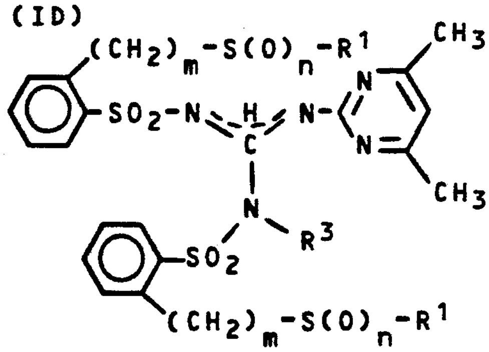

(ID)

in welcher

$R^1$, $R^3$, m und n die oben angegebenen Bedeutungen haben,

mit Aminoverbindungen der Formel (IV)

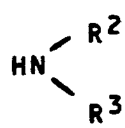

(IV)

in welcher

$R^2$ die vorausgehend unter (b) angegebene Bedeutung hat und

0 173 319

$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder

(c) für den Fall, daß m für Null steht, n für 2 steht,

$R^1$ für $C_1$-$C_4$-Alkoxyamino oder Benzyloxyamino steht und

$R^2$ für Wasserstoff, $C_1$-$C_6$-Alkyl [welches gegebenenfalls wie oben angegeben substituiert ist], $C_3$-$C_6$-Cycloalkyl, $C_3$-$C_6$-Alkenyl oder Benzyl [welches gegebenenfalls wie oben angegeben substituiert ist] steht oder zusammen mit $R^3$ die oben angegebene Bedeutung hat,

wenn man Verbindungen der Formel (V)

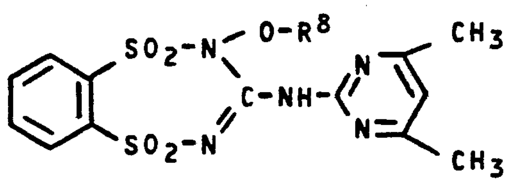

(V)

in welcher

$R^8$ für $C_1$-$C_8$-Alkyl oder Benzyl steht,

mit Aminoverbindungen der Formel (VI)

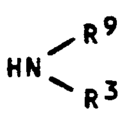

(VI)

in welcher

$R^9$ die oben unter (c) für $R^2$ angegebene Bedeutung hat und

$R^3$ die oben angegebene Bedeutung hat,

gegebenenfalls in Gegenwart von Verdünnungsmitteln umsetzt; oder

(d) für den Fall, daß M für ein Metalläquivalent steht, wenn man die nach den oben unter (a), (b) und (c) angegebenen Verfahren erhältlichen Verbindungen der Formel (I), in welcher M für Wasserstoff steht und $R^1$, $R^2$, $R^3$, m und n die oben angegebenen Bedeutungen haben,

mit Metall-hydroxiden, -hydriden oder -alkanolaten oder mit metallorganischen Verbindungen gegebenenfalls in Gegenwart von Verdünnungsmitteln umsetzt;

oder

(e) für dem Fall, daß 1:1-Addukte von Verbindungen der Formel (I) mit starken Säuren herzustellen sind,

wenn man Verbindungen der Formel (I) in welcher M für Wasserstoff steht und $R^1$, $R^2$, $R^3$, m und n die oben angegebenen Bedeutungen haben,

mit starken Säuren gegebenenfalls in Gegenwart von Verdünnungsmitteln umsetzt.

Die neuen substituierten Arylsulfonylguanidine der Formel (I) und ihre Addukte mit starken Säuren zeichnen sich durch starke herbizide Wirksamkeit aus.

Ueberraschenderweise zeigen die neuen Verbindungen der Formel (I) erheblich bessere herbizide Wirkung als vorbekannte Guanidin-Derivate gleicher Wirkungsrichtung.

Gegenstand der Erfindung sind vorzugsweise Verbindungen der Formel (I), in welcher

m für Null oder 1 steht,

n für Null, 1 oder 2 steht,

M für Wasserstoff oder ein Natrium-, Kalium- oder Calcium-äquivalent steht,

$R^1$ für $C_1$-$C_4$-Alkyl [welches gegebenenfalls durch Fluor und/oder Chlor substituiert ist], Amino, $C_1$-$C_4$-Alkylamino, $C_1$-$C_8$-Alkoxyamino, Di-($C_1$-$C_3$-alkyl)-amino, $C_1$-$C_4$-Alkoxy-$C_1$-$C_3$-alkylamino oder Dimethylhydrazino steht,

$R^2$ für Wasserstoff, $C_1$-$C_4$-Alkyl [welches gegebenenfalls durch Fluor, Chlor, Brom, Cyano, Hydroxy oder $C_1$-$C_2$-Alkoxy substituiert ist], $C_3$-$C_6$-Cycloalkyl, $C_3$-$C_6$-Alkenyl, $C_3$-$C_6$-Alkinyl, Benzyl oder den Rest 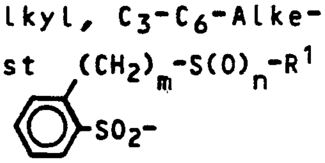

steht, worin
$R^1$, m und n die vorausgehend angegebenen Bedeutungen haben;
in welcher weiter

$R^3$ für Wasserstoff, $C_1$-$C_4$-Alkyl, (welches gegebenenfalls durch Fluor, Chlor, Brom, Cyano, Carboxy, $C_1$-$C_3$-Alkoxy-carbonyl, Hydroxy oder $C_1$-$C_2$-Alkoxy substituiert ist], $C_3$-$C_6$-Cycloalkyl, $C_3$-$C_6$-Alkenyl, $C_3$-$C_6$-Alkinyl, Phenylethyl oder Benzyl [welches gegebenenfalls durch Fluor, Chlor, Nitro, Cyano, $C_1$-$C_2$-Alkyl, $C_1$-$C_2$-Alkoxy oder $C_1$-$C_2$-Alkoxycarbonyl substituiert ist] oder für Phenyl[welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_2$-Alkoxycarbonyl substituiert ist] steht, oder

$R^2$ und $R^3$ gemeinsam für $C_4$-$C_5$-Alkandiyl stehen, welches gegebenenfalls durch eine Sauerstoff-Brücke oder durch eine Brücke >N-$R^4$ unterbrochen ist, wobei

$R^4$ für $C_1$-$C_3$-Alkyl, $C_1$-$C_3$-Alkyl-carbonyl oder Phenyl steht [welches gegebenenfalls durch Fluor, Chlor, Brom, Cyano, Nitro, $C_1$-$C_2$-Alkyl, Trifluormethyl oder $C_1$-$C_2$-Alkoxy substituiert ist];

in welcher weiter

$R^3$ für den Rest -O-$R^5$ steht, worin

$R^5$ für $C_1$-$C_8$-Alkyl [welches gegebenenfalls durch Fluor oder Chlor substituiert ist], $C_3$-$C_6$-Alkenyl, $C_1$-$C_3$-Alkoxy-carbonyl-$C_1$-$C_2$-alkyl, Phenyl, Phenylethyl oder Benzyl [welches gegebenenfalls durch Fluor, Chlor, Nitro, Cyano, $C_1$-$C_2$-Alkyl, $C_1$-$C_2$-Alkoxy oder $C_1$-$C_2$-Alkoxycarbonyl substituiert ist] steht;

in welcher weiter

$R^3$ für den Rest $-N<^{R^6}_{R^7}$ steht, worin

$R^6$ für Wasserstoff oder Methyl steht und

$R^7$ für $C_1$-$C_2$-Alkyl [welches gegebenenfalls durch Fluor, Chlor, Cyano, $C_1$-$C_2$-Alkoxy oder $C_1$-$C_2$-Alkoxycarbonyl substituiert ist], $C_3$-$C_6$-Cycloalkyl, Phenylethyl, Benzyl oder Phenyl [welches gegebenenfalls durch Fluor, Chlor, Brom, Nitro, Cyano, $C_1$-$C_2$-Alkyl, $C_1$-$C_2$-Alkoxy oder $C_1$-$C_2$-Alkoxy-carbonyl substituiert ist] steht.

Gegenstand der Erfindung sind vorzugsweise ferner 1:1-Addukte von Verbindungen der Formel (I) - wie vorausgehend definiert, wobei M für Wasserstoff steht - mit Halogenwasserstoffsäuren, wie Hydrogenchlorid, Hydrogenbromid und Hydrogeniodid, mit Schwefelsäure, Trifluoressigsäure, mit gegebenenfalls durch Fluor oder Chlor substituierten Alkansulfonsäuren mit bis zu 4 Kohlenstoffatomen oder auch mit Benzol- oder Naphthalin-sulfonsäuren, welche gegebenenfalls durch Fluor, Chlor, Brom oder Methyl substituiert sind.

Gegenstand der Erfindung sind insbesondere Verbindungen der Formel (I), in welcher

(A) m für Null oder 1 steht,

n für Null, 1 oder 2 steht,

M für Wasserstoff oder ein Natrium-, Kalium- oder Calcium-äquivalent steht,

$R^1$ für $C_1$-$C_3$-Alkyl, durch Fluor substituiertes $C_1$-$C_3$-Alkyl und - für den Fall, daß n für 2 steht - auch für Di-($C_1$-$C_2$-alkyl)-amino oder $C_1$-$C_4$-Alkoxy-methylamino steht,

$R^2$ für den Rest 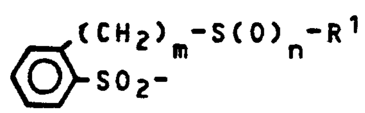 steht, worin

$R^1$, m und n die vorausgehend angegebenen Bedeutungen haben;

in welcher weiter

$R^3$ für den Rest $-O-R^5$ steht, worin
$R^5$ für $C_1-C_8$-Alkyl [welches gegebenenfalls durch Fluor oder Chlor substituiert ist], $C_3-C_5$-Alkenyl, $C_1-C_3$-Alkoxy-carbonylmethyl, Phenyl, Phenylethyl oder Benzyl [welches gegebenenfalls durch Fluor, Chlor, Nitro, Cyano, Methyl, Methoxy, Methoxycarbonyl oder Ethoxycarbonyl substituiert ist) steht,

oder in welcher

(B) m für Null steht,

n für 2 steht,

M für Wasserstoff oder ein Natrium-, Kalium- oder Calcium-äquivalent steht,

$R^1$ für $C_1-C_3$-Alkyl, durch Fluor substituiertes $C_1-C_3$-Alkyl, Amino, $C_1-C_3$-Alkylamino, $C_1-C_3$-Alkoxyamino, Di-($C_1-C_2$-alkyl)-amino, $C_1-C_4$-Alkoxymethylamino oder Dimethylhydrazino steht,

$R^2$ für Wasserstoff steht und

$R^3$ für den Rest $-O-R^5$ steht, worin
$R^5$ für $C_1-C_8$-Alkyl [welches gegebenenfalls durch Fluor oder Chlor substituiert ist], $C_3-C_5$-Alkenyl, $C_1-C_3$-Alkoxy-carbonylmethyl, Phenyl, Phenylethyl oder Benzyl [welches gegebenenfalls durch Fluor, Chlor, Nitro, Cyano, Methyl, Methoxy, Methoxycarbonyl oder Ethoxycarbonyl substituiert ist) steht,

sowie - für den Fall, daß M für Wasserstoff steht - die 1:1-Addukte der vorausgehend definierten Verbindungen mit Salzsäure, Schwefelsäure, Trifluoressigsäure, Methan-

sulfonsäure, Benzolsulfonsäure und p-Toluolsulfonsäure.

Verwendet man beispielsweise für die Verfahrensvariante (a) 2-Ethylsulfonyl-benzolsulfonsäurechlorid und N'-(4,6-Dimethyl-pyrimidin-2-yl)-N''-allyloxy-guanidin als Ausgangsstoffe, so kann der Reaktionsablauf durch folgendes Formelschema skizziert werden:

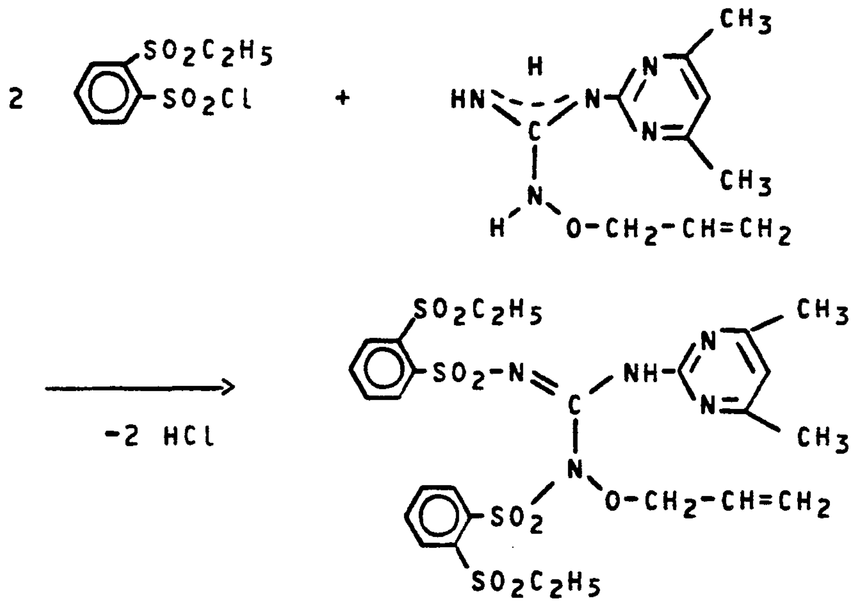


Verwendet man beispielsweise für die Verfahrensvariante (b) N'-(4,6-Dimethyl-pyrimidin-2-yl)-N''-methoxy-N'',N'''-bis-(2-diethylaminosulfonyl-benzolsulfonyl)-guanidin und Benzyloxyamin als Ausgangsstoffe, so kann der Reaktionsablauf durch folgendes Formelschema skizziert werden:

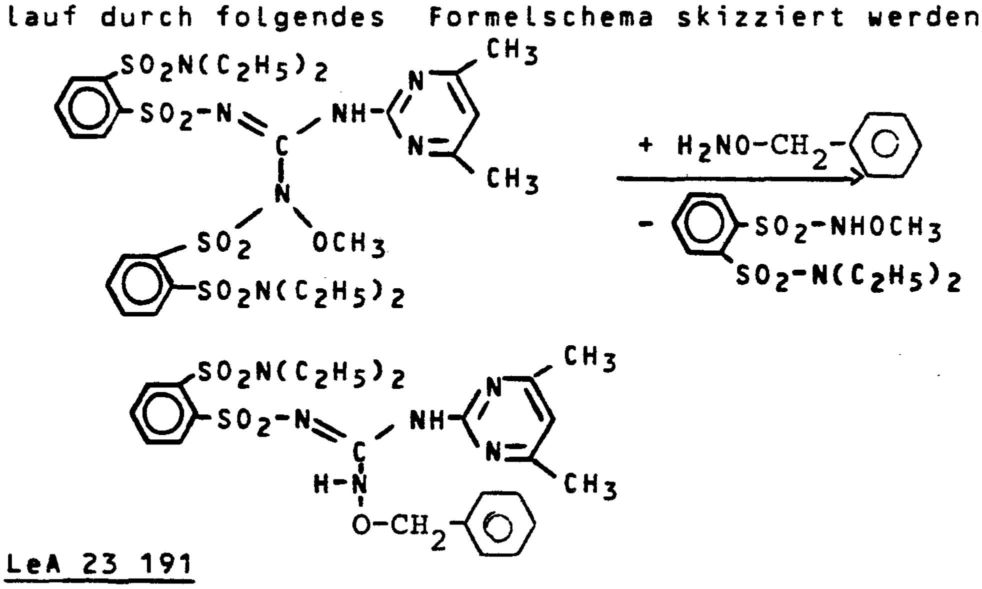

Verwendet man beispielsweise für die Verfahrensvariante (c) das nachstehend angegebene Benzodisultam und N,N-Dimethylhydrazin als Ausgangsstoffe, so kann der Reaktionsablauf durch folgendes Formelschema skizziert werden:

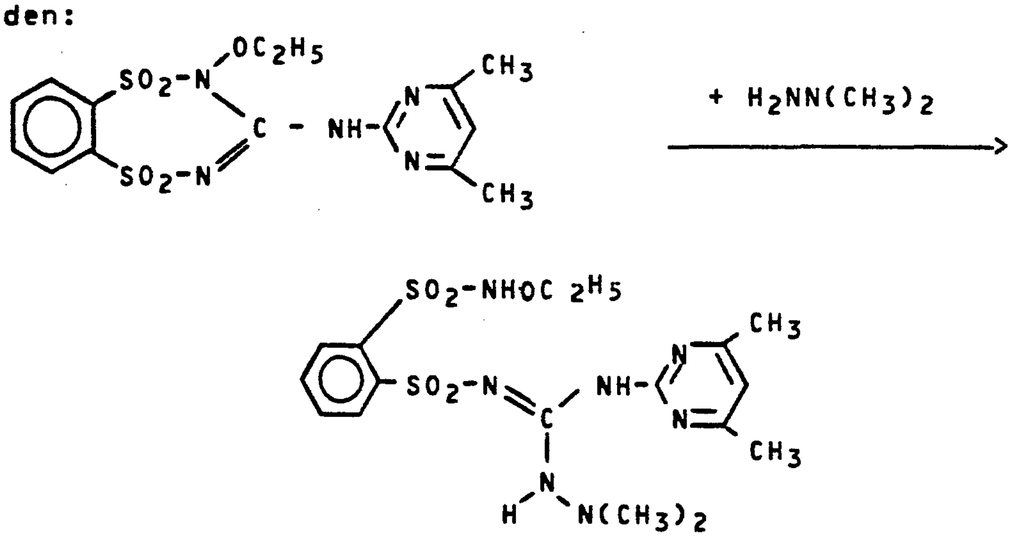


Verwendet man beispielsweise für die Verfahrensvariante (d) N'-(4,6-Dimethyl-pyrimidin-2-yl)-N''-phenoxy-N'''-(2-methylthiomethyl-benzolsulfonyl)-guanidin und Kaliumethanolat als Ausgangsstoffe, so kann der Reaktionsablauf durch das folgende Formelschema skizziert werden:

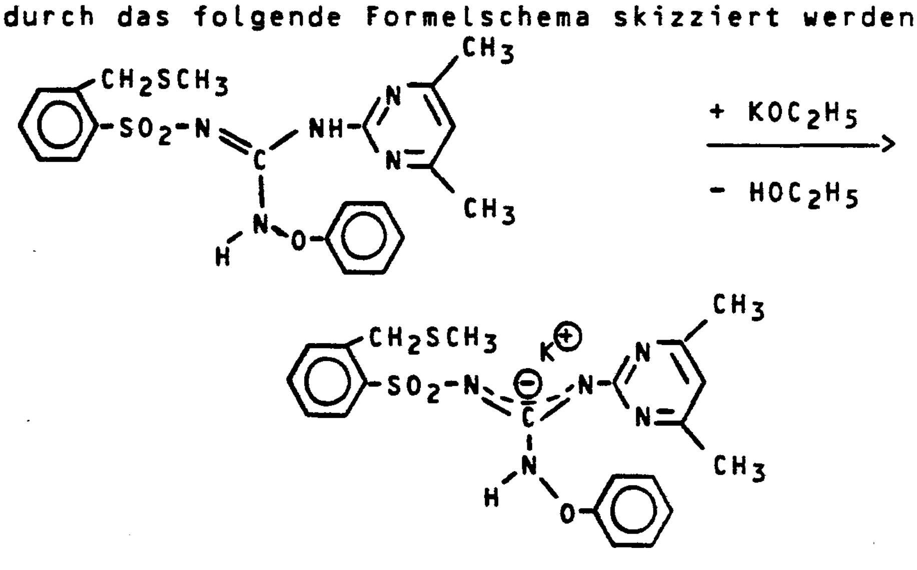

Verwendet man beispielsweise für die Verfahrensvariante (e) N'-(4,6-Dimethyl-pyrimidin-2-yl)-N''-methoxy-N'',N'''-bis-(2-propylthio-benzolsulfonyl)-guanidin und Salzsäure als Ausgangsstoffe, so kann der Reaktionsablauf durch folgendes Formelschema skizziert werden:

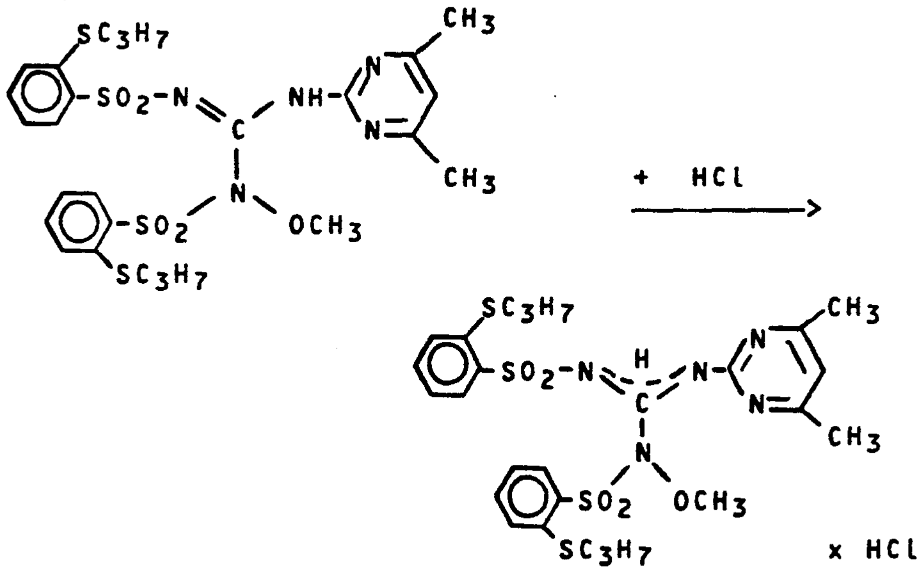

Die als Ausgangsstoffe für die Verfahrensvariante (a) zu verwendenden Guanidin-Derivate sind durch die Formel (II) allgemein definiert. In Formel (II) hat $R^3$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Ausgangsstoffe der Formel (II) seien beispielsweise genannt: N'-(4,6-Dimethyl-pyrimidin-2-yl)-N''-methoxy-guanidin, -N''-ethoxy-guanidin, -N''-propoxy-guanidin, -N''-isopropoxy-guanidin, -N''-butoxy-guanidin, -N''-isobutoxy-guanidin, -N''-sec.-butoxy-guanidin, -N''-pentoxy-guanidin, -N''-isopentoxy-guanidin, -N''-sec.-pentoxy-guanidin, -N''-hexyloxy-guanidin, -N''-isohexyloxy-guanidin, -N''-heptyloxy-guanidin, -N''-isoheptyloxy-guanidin, -N''-octyloxy-guanidin, -N''-isooctyloxy-guanidin,

-N''-allyloxy-guanidin, -N''-crotyloxy-guanidin, -N''-(2-chlor-ethoxy)-guanidin, -N''-(2-fluor-ethoxy)-guanidin, -N''-(2-chlor-propoxy)-guanidin, -N''-(3-chlor-propoxy)-guanidin, -N''-(4-chlor-butoxy)-guanidin, -N''-methoxy-carbonylmethyloxy-guanidin, -N''-ethoxycarbonylmethoxy-guanidin, -N''-(1-methoxycarbonyl-ethoxy)-guanidin, -N''-(1-ethoxy-carbonyl-ethoxy)-guanidin, -N''-Aminocarbonyl-methoxy-guanidin, -N''-(phenyl-ethoxy)-guanidin, -N''-phenoxy-guanidin, -N''-(4-methyl-benzyloxy)-guanidin, -N''-(4-chlor-benzyloxy)-guanidin, -N''-(4-nitro-benzyloxy)-guanidin, -N''-(2,6-dichlor-benzyloxy)-guanidin, -N''(4-ethoxy-carbonyl-benzyloxy)-guanidin und -N''-(4-methoxycarbonyl-benzyloxy)-guanidin.

Die Ausgangsstoffe der Formel (II) sind teilweise bekannt (vergl. J. Chem. Soc. 1962, 3915); zum Teil sind sie Gegenstand einer nicht zum vorveröffentlichten Stand der Technik gehörenden Patentanmeldung der Anmelderin (vergl. DE-OS 3 334 455).

Man erhält die Verbindungen der Formel (II), wenn man 2-Cyanamino-4,6-dimethyl-pyrimidin der Formel (VII)

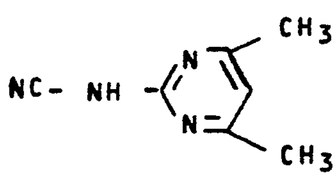

(VII)

mit Aminoverbindungen der Formel (VIII)

$$H_2N - R^3 \qquad (VIII)$$

in welcher

$R^3$ die oben angegebene Bedeutung hat

bzw. mit deren Hydrochloriden,

gegebenenfalls in Gegenwart von Verdünnungsmitteln, wie z.B. Ethanol, Isopropanol oder Butanol, bei Temperaturen zwischen 20°C und 150°C, vorzugsweise zwischen 50°C und 120°C, umsetzt und gegebenenfalls die Umsetzungsprodukte mit Säureakzeptoren, wie z.B. Ammoniak, Natronlauge oder Soda behandelt.
2-Cyanamino-4,6-dimethyl-pyrimidin der Formel (VII)ist bereits bekannt (vgl. J.Chem.Soc. 1953, 1725).

Die Aminoverbindungen der Formel (VIII)sind ebenfalls bereits bekannt und können nach an sich bekannten Verfahren hergestellt werden (vgl. Chem.Pharm. Bull. 15, (1967), 345; Bull. Soc. Chim. France 1958, 664; Synthesis 1976, 682; J.Chem.Soc. 1930, 228 und Helv. Chim. Acta 45 (1962),1387).

Die weiter als Ausgangsstoffe für Verfahren (a) zu verwendenden substituierten Arensulfonsäurechloride sind durch die Formel (III) allgemein definiert. In Formel (III) haben $R^1$ sowie m und n vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (III) seien beispielsweise genannt: 2-Methylthio-, 2-Ethylthio-, 2-Propylthio-, 2-Isopropylthio-, 2-Butylthio-, 2-Isobutylthio-, 2-Difluormethylthio-, 2-Trifluormethylthio-, 2-Methylsulfinyl-, 2-Ethylsulfinyl-, 2-Propylsulfinyl-, 2-Isopropylsulfinyl-, 2-Butylsulfinyl-, 2-Isobutylsulfinyl-, 2-Methylsulfonyl-, 2-Ethylsulfonyl-, 2-Propylsulfonyl-, 2-Isopropylsulfonyl-, 2-Butylsulfonyl-, 2-Trifluormethylsulfonyl-, 2-Dimethylaminosulfonyl-, 2-Diethylaminosulfonyl-, 2-N-Methoxy-N-methylaminosulfonyl-, 2-Methylthiomethyl-, 2-Methylsulfinylmethyl-, 2-Methylsulfonylmethyl-, 2-Ethylthiomethyl-, 2-Propylthiomethyl- und 2-Isopropylthiomethyl-benzolsulfonsäurechlorid.

Die Verbindungen der Formel (III) sind bekannt und können nach an sich bekannten Verfahren hergestellt werden (vgl. EP-OS 23 140, 35 893 und 72 347, US-PS 4 348 220 und 4 397 679).

Die als Ausgangsstoffe für die Verfahrensvariante (b) zu verwendenden substituierten Arylsulfonylguanidine sind durch die Formel (ID) allgemein definiert. In Formel (ID) haben $R^1$ und $R^3$ sowie m und n vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (ID) seien genannt:
N'-(4,6-Dimethyl-pyrimidin-2-yl)-N''-methoxy-N'',N'''-bis-(2-methylthio-benzolsulfonyl)- und N'-(4,6-Dimethyl-pyrimidin-2-yl)-N''-methoxy-N'',N'''-bis-(2-dimethylaminosulfonyl-benzolsulfonyl)-guanidin.

Die Verbindungen der Formel (ID) können nach dem oben unter (a) beschriebenen Verfahren hergestellt werden.

Die bei Verfahrensvariante (b) weiter als Ausgangsstoffe zu verwendenden Aminoverbindungen sind durch die Formel (IV) allgemein definiert. In Formel (IV) haben $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (IV) seien beispielsweise genannt:

O-Propyl-, O-Isopropyl-, O-Butyl-, O-Isobutyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl-, O-Crotyl-, O-(2-Chlor-ethyl)-, O-(2-Fluor-ethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4-Fluor-benzyl)-, O-(4-Chlor-benzyl)-, O-(4-Nitro-benzyl)-, O-(2,6-Dichlor-benzyl)-, O-(4-Methoxycarbonyl-benzyl)- und O-(4-Ethoxy-carbonyl-benzyl)-hydroxylamin.

Aminoverbindungen der Formel (IV) sind bekannt und können nach an sich bekannten Verfahren hergestellt werden(vgl.Chem. Pharm. Bull. 15 (1967), 345; Bull. Soc. Chim. France 1958, 664; Synthesis 1976, 682; J.Chem. Soc.1930, 228 und Helv. Chim. Acta 45 (1962), 1387).

Die als Ausgangsstoffe für die Verfahrensvariante (c) zu verwendenden Verbindungen sind durch die Formel (V) allgemein definiert. In Formel (V) hat $R^3$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Beispiele seien Verbindungen der Formel (V) genannt, in welcher $OR^8$ für Methoxy, Ethoxy, Propoxy, Isopropoxy, Butoxy, Isobutoxy, sec.-Butoxy, Octyloxy, Benzyloxy, Allyloxy, 3-Chlorpropyloxy, Methoxycarbonylmethoxy, 2-Phenylethoxy, 4-Methyl-benzyloxy, 4-Fluorbenzyloxy, 4-Chlor-benzyloxy, 4-Nitro-benzyloxy oder 4-Methoxycarbonyl-benzyloxy oder 4-Ethoxycarbonyl-benzyloxy steht.

Die Verbindungen der Formel (V) sind noch nicht in der Literatur beschrieben. Man erhält die neuen Verbindungen der Formel (V), wenn man Guanidin-Derivate der Formel (II)

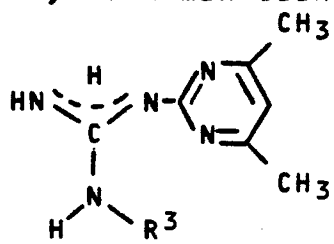

(II)

in welcher

$R^3$ die oben angegebene Bedeutung hat,

mit Benzol-1,2-disulfonsäure-dichlorid der Formel (IX)

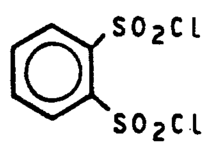

(IX)

gegebenenfalls in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid oder Chloroform, bei Temperaturen zwischen -20°C und +30°C umsetzt.

Die Herstellung der Guanidin-Derivate der Formel (II) wurde bereits weiter oben im Zusammenhang mit dem unter (a) angegebenen Verfahren beschrieben.

Benzol-1,2-disulfonsäure-dichlorid der Formel (IX) ist bekannt (vgl. J.Org.Chem. 31/1966), 3289-3292).

Die bei Verfahren (c) weiter als Ausgangsstoffe zu verwendenden Aminoverbindungen sind durch die Formel (VI) allgemein definiert. In Formel (VI) stehen

$R^9$ vorzugsweise für Wasserstoff oder $C_1$-$C_3$-Alkyl, insbesondere für Wasserstoff, Methyl oder Ethyl, und

$R^3$ vorzugsweise für Wasserstoff, $C_1$-$C_4$-Alkyl, $C_1$-$C_4$-Alkoxy oder Dimethylamino, insbesondere für Wasserstoff, Methyl, Ethyl, Methoxy, Ethoxy oder Propoxy.

Als Beispiele für die Aminoverbindungen der Formel (VI) seien genannt:
Ammoniak, Methylamin, Ethylamin, Propylamin, Isopropylamin, Butylamin, Isobutylamin, Dimethylamin, Diethylamin, Dimethylhydrazin, O-Methyl-, O-Ethyl-, O-Propyl-, O-Isopropyl-, O-Butyl- und O-Isobutylhydroxylamin sowie N,O-Dimethylhydroxylamin.

Die Verbindungen der Formel (VI) sind bekannt und können nach an sich bekannten Verfahren hergestellt werden (vgl. Chem. Pharm. Bull. 15 (1967), 345).

Die bei der Verfahrensvariante (d) als Ausgangsstoffe zu verwendenden substituierten Arylsulfonylguanidine sind durch die Formel (I) - mit der Maßgabe, daß M für Wasserstoff steht - allgemein definiert. In Formel (I) - soweit sie die als Ausgangsstoffe für Verfahren (d) zu verwendenden Verbindungen betrifft - steht M für Wasserstoff und die Reste $R^1$, $R^2$ und $R^3$ sowie m und n haben vorzugsweise bzw. insbesondere die gleichen Bedeutungen, wie sie oben im Rahmen der Substituentendefinition für Formel (I) vorzugsweise bzw. als insbesondere bevorzugt angegeben sind.

Die als Ausgangsstoffe für Verfahren (d) zu verwendenden Verbindungen der Formel (I) können nach den oben unter (a), (b) und (c) beschriebenen Verfahren hergestellt werden.

Als Beispiele für die bei Verfahren (d) zu verwendenden Metallhydroxide, -hydride, -alkanolate bzw. metallorganischen Verbindungen seien genannt: Lithium-, Natrium-, Kalium-, Magnesium- und Calcium-hydroxid, Lithium-, Natrium- und Calcium-hydrid, Natrium-methanolat und -ethanolat, Kalium-methanolat, -ethanolat und Kalium-tert.-butanolat sowie Butyllithium und Isopropylmagnesiumchlorid.

Die bei Verfahren (e) als Ausgangsstoffe zu verwendenden substituierten Arylsulfonylguanidine sind durch die Formel (I) - mit der Maßgabe, daß M für Wasserstoff steht - allgemein definiert. In Formel (I) - soweit sie die als Ausgangsstoffe für Verfahren (e) zu verwendenden Verbindungen betrifft - steht M für Wasserstoff und die Reste $R^1$, $R^2$ und $R^3$ sowie m und n haben vorzugsweise bzw. insbesondere die gleichen Bedeutungen, wie sie oben im Rahmen der Substituentendefinition der Formel (I) vorzugsweise bzw. als insbesondere bevorzugt angegeben sind.

Die als Ausgangsstoffe für Verfahren (e) zu verwendenden Verbindungen der Formel (I) können nach den oben unter (a), (b) und (c) beschriebenen Verfahren hergestellt werden.
Bei Verfahren (e) werden starke Säuren als Ausgangsstoffe eingesetzt. Vorzugsweise sind dies Halogenwasserstoffsäuren, wie Hydrogenchlorid, Hydrogenbromid, oder Hydrogeniodid, weiter Schwefelsäure oder gegebenenfalls durch Fluor oder Chlor substituierte Alkansulfonsäuren mit bis zu 4 Kohlenstoffatomen, wie z.B. Methansulfonsäure, 2-Chlorethansulfonsäure und Trifluormethansulfonsäure, Trifluoressigsäure, ferner Benzolsulfonsäure, p-Toluolsulfonsäure, Naphthalin-1,4-, -1,5-, -1,6-, -2,6- und -2,7-disulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls substituierte Kohlenwasserstoffe, wie z.B. Methylenchlorid, Chloroform, 1,2-Dichlorethan, Toluol, Xylol und Chlorbenzol, Nitrile, wie z.B. Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.
Als Säureakzeptoren können bei Verfahren (a) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80 und +100°C, vorzugsweise zwischen -30 und +50°C. Das erfindungsgemäße Verfahren (a) wird im allgemeinen bei Normaldruck durchgeführt.

Zur Durchführung von Verfahren (a) setzt man je Mol Guanidin-Derivat der Formel (II) im allgemeinen zwischen 2 und 5 Mol, vorzugsweise zwischen 2,1 und 3 Mol substituiertes Arensulfonsäurechlorid der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden: Gegebenenfalls nach Abdestillieren flüchtiger Komponenten wird mit Wasser und einem mit Wasser nicht mischbaren Lösungsmittel,wie z.B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z.B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol,Ethanol, n- und i-Propanol, Ether, wie Tetrahydrofuran, Dioxan und 1,2-Dimethoxyethan, Ester,wie Essigsäuremethylester und -ethylester, Nitrile, wie z.B.Acetonitril oder Propionitril, sowie Dimethylformamid und Wasser.

Als Säureakzeptoren können bei Verfahren (b) Säurebindemittel eingesetzt werden, welche in ihren nucleophilen Eigenschaften nicht nennenswert mit den Aminoverbindungen der Formel (IV) konkurrieren.
Als solche seien Alkalimetall- und Erdalkalimetallcarbonate, wie z.B. Kaliumcarbonat und Calciumcarbonat, tertiäre Amine, wie z.B. Triethylamin, N,N-Dimethylanilin und N,N-Dimethylbenzylamin, sowie Stickstoffheterocyclen, wie z.B. Pyridin, Diazabicyclooctan (DABCO) und Diazabicycloundecen (DBU) genannt.

Die Reaktionstemperatur kann bei Verfahren (b) innerhalb eines größeren Bereiches variiert werden. Im allgemeinen arbeitet man zwischen 0°C und 150°C, vorzugsweise zwischen 10°C und 100°C. Verfahren (b) wird im allgemeinen bei Normaldruck durchgeführt.

Zur Durchführung des erfindungsgemäßen Verfahrens (b) setzt man je Mol Verbindung der Formel (ID) im allgemeinen zwischen 1 und 10 Mol, vorzugsweise zwischen 2 und 5 Mol Aminoverbindung der Formel (IV) oder deren Hydrochlorid ein.
Im allgemeinen wird das substituierte Arylsulfonylguanidin der Formel (ID) mit dem Verdünnungsmittel bei Raumtemperatur oder unter leichtem Kühlen vorgelegt und die Aminoverbindung der Formel (IV) bzw. das Hydrochlorid hiervon und gegebenenfalls ein geeigneter Säureakzeptor dazugegeben. Das Reaktionsgemisch wird dann im allgemeinen bei Raumtemperatur oder erhöhter Temperatur bis zum Reaktionsende gerührt.

Die Aufarbeitung kann nach üblichen Methoden durchgeführt werden. Soweit die Produkte der Formel (I) aus dem Reaktionsgemisch kristallin anfallen, können sie durch Absaugen isoliert werden. Anderenfalls wird - gegebenenfalls nach Einengen - mit Wasser verdünnt und mit einem mit Wasser praktisch nicht mischbaren Lösungsmittel, wie z.B. Methylenchlorid, extrahiert. Durch Waschen der Extraktionslösung mit Wasser, Trocknen, Filtrieren, Einengen des Filtrates und Umkristallisieren des Rückstandes können die Produkte der Formel (I) in reiner Form erhalten werden.

Das erfindungsgemäße Verfahren (c) zur Herstellung der neuen Verbindungen der Formel (I) wird vorzugsweise unter Verwendung von Verdünnungsmitteln durchgeführt. Als solche kommen praktisch alle inerten organischen Lösungsmittel in Betracht, wie sie oben im Zusammenhang mit der Beschreibung des erfindungsgemäßen Verfahrens (b) genannt wurden.

Die Reaktionstemperatur kann bei Verfahren (c) innerhalb eines größeren Bereiches variiert werden. Im allgemeinen arbeitet man zwischen -20°C und +50°C, vorzugsweise zwischen 0°C und 30°C. Verfahren (c) wird im allgemeinen bei Normaldruck durchgeführt.

Zur Durchführung von Verfahren (c) setzt man je Mol Verbindung der Formel (V) im allgemeinen zwischen 1 und 3 Mol, vorzugsweise zwischen 1 und 2 Mol Aminoverbindung der Formel (VI) ein.
Im allgemeinen wird die Verbindung der Formel (V) mit dem Verdünnungsmittel vorgelegt und die Aminoverbindung der Formel (VI) - gegebenenfalls im Verdünnungsmittel gelöst - zudosiert. Das Reaktionsgemisch wird bis zum Reaktionsende gerührt. Die Aufarbeitung kann nach üblichen Methoden durchgeführt werden.Im allgemeinen erhält man die Produkte der Formel (I) nach Filtration, Einengen des Filtrats und Verreiben des Rückstandes mit Alkohol in kristalliner Form.

Das erfindungsgemäße Verfahren (d) wird vorzugsweise unter Verwendung von Verdünnungsmitteln durchgeführt. Als solche kommen praktisch alle inerten organischen Lösungsmittel in Betracht. Hierzu gehören insbesondere Alkohole,

wie z.B. Ethanol, n- und iso-Propanol, Ether, wie z.B. Tetrahydrofuran, Dioxan und 1,2-Dimethoxyethan, Ester, wie z.B. Essigsäureethylester und-methylester sowie Nitrile, wie z.B. Acetonitril.

Die Reaktionstemperatur kann bei Verfahren (d) innerhalb eines größeren Bereiches variiert werden. Im allgemeinen arbeitet man zwischen -20°C und +50°C, vorzugsweise zwischen 0°C und 30°C. Verfahren (d) wird im allgemeinen bei Normaldruck durchgeführt.

Zur Duchführung des erfindungsgemäßen Verfahrens (d) setzt man je Mol Verbindung der Formel (I) im allgemeinen zwischen 0,9 und 1,2 Mol, vorzugsweise zwischen 0,95 und 1,1 Mol Metallverbindung ein.

Im allgemeinen werden die Verbindungen der Formel (I) und das Verdünnungsmittel vorgelegt und - gegebenenfalls unter leichter Außenkühlung - die Metallverbindung - gegebenenfalls im Verdünnungsmittel gelöst - zudosiert.Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (e) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester sowie Ketone, wie Aceton, Methylethylketon und Methylisobutylketon.

Soweit die als Ausgangsstoffe verwendeten Säuren im wässriger Lösung eingesetzt werden, kann vorteilhaft auch Essigsäureanhydrid als Verdünnungsmittel verwendet werden.

Die Reaktionstemperatur kann bei Verfahren (e) innerhalb eines größeren Bereiches variiert werden. Im allgemeinen arbeitet man zwischen -20°C und +50°C, vorzugsweise zwischen 0°C und 30°C. Verfahren (e) wird im allgemeinen bei Normaldruck durchgeführt.

Zur Durchführung des erfindungsgemäßen Verfahrens (e) setzt man je Mol Verbindung der Formel (I) im allgemeinen zwischen 1 und 10 Mol, vorzugsweise 1 und 5 Mol einer starken Säure ein.

Im allgemeinen werden die Verbindungen der Formel (I) und das Verdünnungsmittel vorgelegt und - gegebenenfalls unter leichter Außenkühlung - die starke Säure zudosiert. Das Reaktionsgemisch wird bis zum Reaktionsende gerührt. Die 1:1-Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne

Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur Bekämpfung monokotyler und dikotyler Unkräuter in monokotylen und dikotylen Kulturen im Vorauflauf- und im Nachauflauf-Verfahren eingesetzt werden.
Eine Kontrolle keimender, auflaufender und bereits etablierter Unkräuter in Dauerkulturen ist mit den erfindungsgemäßen Wirkstoffen ebenso möglich, wie die totale Vegetationskontrolle auf Nichtkulturland.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001und 10 kg Wirkstoff pro Hektar Bodenfläche, vorzugsweise zwischen 0,05 und 5 kg pro ha.
Verbindungen der Formel (I) zeigen auch fungizide Wirkung, so z.B. gegen Pyricularia oryzae am Reis.

Die Herstellung und Verwendung der erfindungsgemäßen Stoffe geht aus den nachfolgenden Beispielen hervor.

Herstellungsbeispiele

Beispiel 1

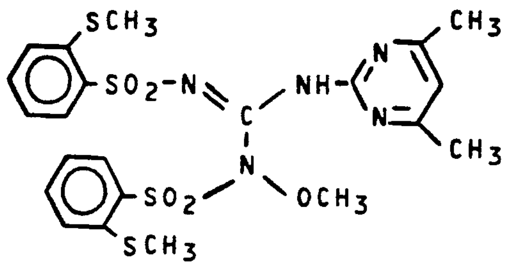

(Verfahren (a))

11,5g (0,05 Mol) 2-Methylthio-benzolsulfonsäurechlorid werden zu einer auf -5°C gekühlten Mischung aus 5,0g (0,025 Mol) N'-(4,6-Dimethyl-pyrimidin-2-yl)-N''-methoxy-guanidin und 50ml Pyridin unter Rühren tropfenweise gegeben. Das Reaktionsgemisch wird eine Stunde bei -5°C und 12 Stunden bei 20°C gerührt und anschließend eingeengt. Der Rückstand wird in 100 ml Methylenchlorid gelöst, diese Lösung mit 2N-Salzsäure und mit Wasser gewaschen, getrocknet, filtriert und eingeengt. Das beim Verreiben des Rückstandes mit Ethanol kristallin angefallene Produkt wird durch Absaugen isoliert.

Man erhält 4,5g ( 32 % der Theorie) N'-(4,6-Dimethyl-pyrimidin-2-yl)-N''-methoxy-N'',N'''-bis-(2-methylthio-benzolsulfonyl)-guanidin vom Schmelzpunkt 155°C.

Beispiel 2

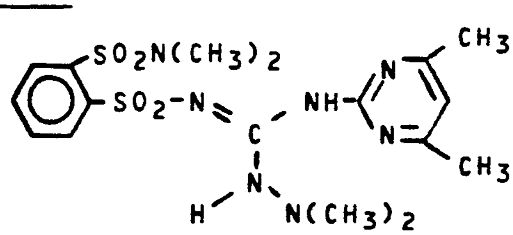

(Verfahren (b))

Eine Mischung aus 7,3g (0,01 Mol) N'-(4,6-Dimethyl-pyrimidin-2-yl)-N''-methoxy-N'',N'''-bis-(2-dimethyl-

aminosulfonyl-benzolsulfonyl)-guanidin, 1,5g (0,025 Mol) N,N-Dimethylhydrazin, 30 ml Ethanol und 5 ml Wasser wird eine Stunde unter Rückfluß zum Sieden erhitzt.
Nach Einengen wird das im Rückstand verbliebene Produkt durch Säulenchromatographie (Hexan/Aceton, 7:3, an Kieselgel) gereinigt.
Man erhält 1,0g (22 % der Theorie) N'-(4,6-Dimethyl-pyrimidin-2-yl)-N''-dimethylamino-N'''-(2-dimethylamino-sulfonyl-benzolsulfonyl)-guanidin vom Schmelzpunkt 184°C.

Beispiel 3

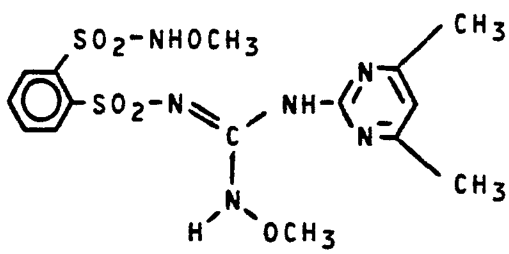

(Verfahren (c))
2,4g (0,05 Mol) O-Methyl-hydroxylamin werden bei 20°C zu einer Mischung aus 18,8g (0,05 Mol) der Verbindung nachstehender Formel

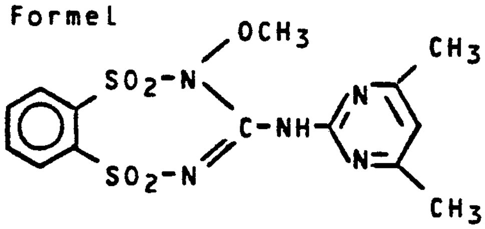

sowie 10g (0,05 Mol) Pyridin und 100 ml Methylenchlorid tropfenweise gegeben. Das Reaktionsgemisch wird 6 Stunden bei 20°C gerührt, anschließend filtriert und eingeengt. Der Rückstand wird mit Ethanol verrieben und das kristallin ausgefallene Produkt durch Absaugen isoliert.
Man erhält 10g (50 % der Theorie) N'-(4,6-Dimethyl-pyrimidin-2-yl)-N''-methoxy-N'''-(2-methoxyaminosulfonyl-benzolsulfonyl)-guanidin vom Schmelzpunkt 208°C(Zers.).

Beispiel 4

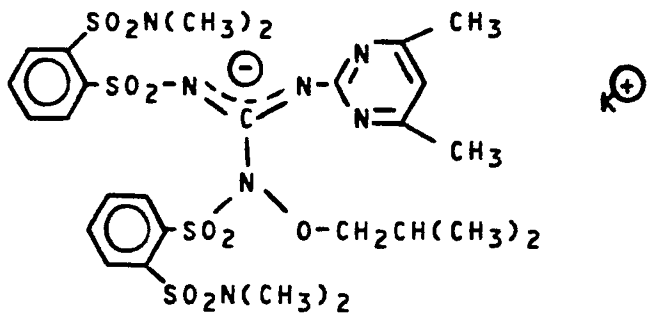

(Verfahren (d))

Eine Mischung aus 7,3g (0,01 Mol) N'-(4,6-Dimethyl-pyrimidin-2-yl)-N''-(2-methyl-propoxy)-N'',N'''-bis-(2-dimethylaminosulfonyl-benzolsulfonyl)-guanidin, 0,9g (0,011 Mol) Kaliumethanolat und 20 ml Ethanol wird 15 Stunden bei 20°C gerührt. Dann wird das kristallin angefallene Produkt durch Absaugen isoliert.

Man erhält 4,1g (53 % der Theorie) N'-(4,6-Dimethyl-pyrimidin-2-yl)-N''-(2-methyl-propoxy)-N'',N'''-bis-(2-dimethylaminosulfonyl-benzolsulfonyl)-guanidin-Kaliumsalz vom Schmelzpunkt 174°C.

Beispiel 5

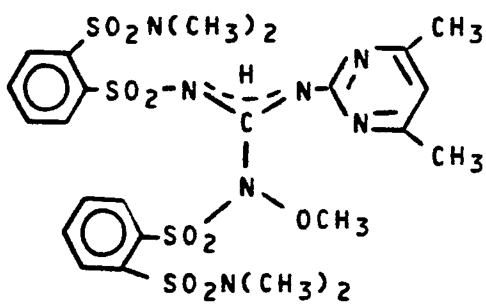

x $CH_3SO_3H$

(Verfahren (e))

Eine Mischung aus 3,5g (0,05 Mol) N'-(4,6-Dimethyl-pyrimidin-2-yl)-N''-methoxy-N'',N'''-bis-(2-dimethylaminosulfonyl-benzolsulfonyl)-guanidin, 0,5 g(0,05 Mol)

Methansulfonsäure und 10 ml Aceton wird 5 Stunden bei 20°C gerührt. Das kristallin angefallene Produkt wird durch Absaugen isoliert. Man erhält 3,4g (87 % der Theorie) des 1:1-Adduktes aus N'-(4,6-Dimethyl-pyrimidin-2-yl)-N''-methoxy-N'',N'''-bis-(2-dimethylaminosulfonyl-benzolsulfonyl)-guanidin und Methansulfonsäure vom Schmelzpunkt 125°C.

Analog erhält man das 1:1-Addukt mit Schwefelsäure vom Schmelzpunkt 177°C (= Beispiel 5a).

Nach den in den vorausgehenden Beispielen exemplarisch beschriebenen Verfahren können die in der nachstehenden Tabelle 1 aufgeführten Verbindungen der Formel (I) hergestellt werden:

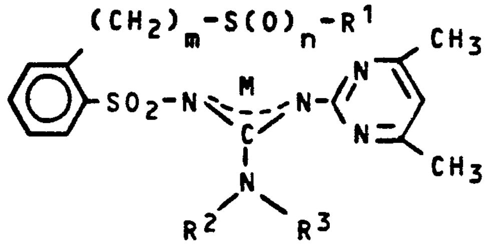

(I)

Tabelle 1

| Bsp. Nr. | m | n | M | $R^1$ | $R^2$ | $R^3$ | Schmelzpunkt(°C) |
|---|---|---|---|---|---|---|---|
| 6 | 0 | 0 | H | $CH_3$ | 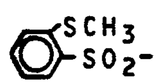 | $-OCH_2-$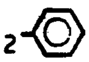 | 146 |
| 7 | 0 | 0 | H | $CH_3$ | 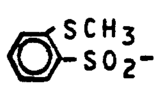 | $-OC_8H_{17}$ | 152 |
| 8 | 0 | 0 | H | $CH_3$ | 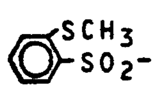 | $-OC_4H_9$-n | 170 |
| 9 | 0 | 2 | H | $-N(CH_3)_2$ | 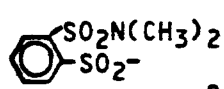 | $-OCH_3$ | 138 |

Tabelle 1-Fortsetzung

| Bsp. Nr. | m | n | M | $R^1$ | $R^2$ | $R^3$ | Schmelzpunkt (°C) |
|---|---|---|---|---|---|---|---|
| 10 | 0 | 0 | H | $CH(CH_3)_2$ | 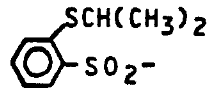 | $-OCH_3$ | (amorph) |
| 11 | 0 | 2 | H | $CH_3$ | 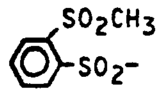 | $-OCH_3$ | 155 |
| 12 | 0 | 2 | H | $-NHOCH_3$ | H | $-N(CH_3)_2$ | 188(Zers.) |
| 13 | 0 | 2 | H | $CH_3$ | 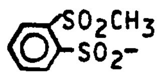 | $-OCH_2-$ 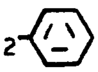 | |
| 14 | 0 | 2 | H | $CH_3$ | 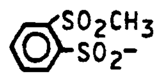 | $-OC_4H_9-n$ | |
| 15 | 1 | 2 | H | $CH_3$ | 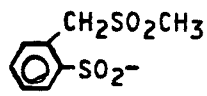 | $-OCH_3$ | |
| 16 | 1 | 2 | H | $CH_3$ | 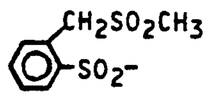 | $-OCH_2COOC_2H_5$ | |
| 17 | 0 | 2 | H | $-N(CH_3)_2$ | 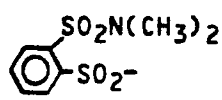 | $-OCH_2CH(CH_3)_2$ | 146 |
| 18 | 0 | 2 | H | $-N(CH_3)_2$ | 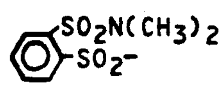 | 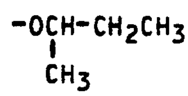 | 149 |
| 19 | 0 | 2 | H | 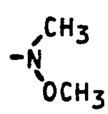 | 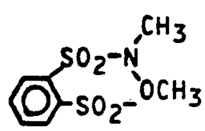 | $-OCH_3$ | |
| 20 | 0 | 2 | H | 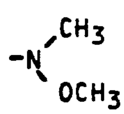 | 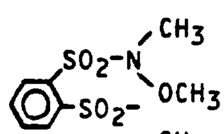 | $-OCH_2CH(CH_3)_2$ | |
| 21 | 0 | 2 | H | 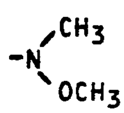 | 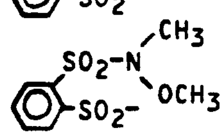 | 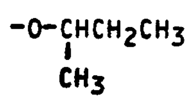 | |
| 22 | 0 | 2 | H | $-N(C_2H_5)_2$ | 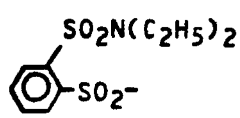 | $-OCH_3$ | 141 |

Tabelle 1 - Fortsetzung

| Bsp. Nr | m | n | M | $R^1$ | $R^2$ | $R^3$ | Schmelz-punkt(°C) |
|---|---|---|---|---|---|---|---|
| 23 | 0 | 2 | H | $-N(C_2H_5)_2$ | H | $-N(CH_3)_2$ | |
| 24 | 0 | 2 | H | 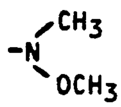 | H | 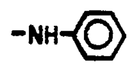 | |
| 25 | 1 | 2 | H | $CH_3$ | H | $-OCH_2COOC_2H_5$ | |
| 26 | 1 | 1 | H | $CH_3$ | 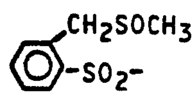 | $-OCH_3$ | |
| 27 | 1 | 1 | H | $CH_3$ | H | $-OCH_2COOCH_3$ | |
| 28 | 1 | 1 | H | $CH_3$ | H | $-N(CH_3)_2$ | |
| 29 | 1 | 1 | H | $CH_3$ | 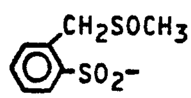 | $-OCH_2COOCH_3$ | |
| 30 | 1 | 2 | H | $CH_3$ | H | $-OCH_2COOC_2H_5$ | |
| 31 | 1 | 2 | H | $CH_3$ | H | $-N(CH_3)_2$ | |
| 32 | 1 | 2 | H | $-CH(CH_3)_2$ | H | $-OCH_3$ | |
| 33 | 0 | 0 | H | $C_2H_5$ | H | $-OCH_2CH=CH_2$ | |
| 34 | 0 | 0 | H | $C_2H_5$ | 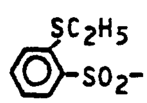 | $-OCH_3$ | |
| 35 | 0 | 2 | H | $CH_3$ | 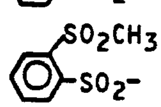 | $-OCH_2CH(CH_3)_2$ | |
| 36 | 0 | 2 | H | $N(CH_3)_2$ | H | $-NHCOCH_3$ | Oel |
| 37 | 0 | 2 | H | $N(CH_3)_2$ | H | 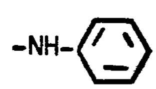 | 164 |

Tabelle 1 (Fortsetzung)

| Bsp. Nr. | m | n | M | $R^1$ | $R^2$ | $R^3$ | Schmelz-punkt ($^oC$) |
|---|---|---|---|---|---|---|---|
| 38 | O | O | H | $-CF_3$ | 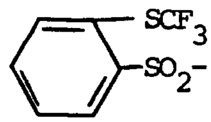 | $-OCH_3$ | 182-183 |
| 39 | O | 2 | H | $-NHOCH_3$ | H | $-OCH_2-$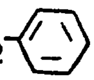 | 154 |
| 40 | O | 2 | H | $-N(C_2H_5)_2$ | H | $-NH_2$ | 182 |
| 41 | O | 2 | H | $-CH_3$ | 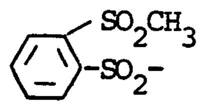 | $-OC_8H_{17}-n$ | amorph |
| 42 | O | 2 | H | $-N(C_2H_5)_2$ | 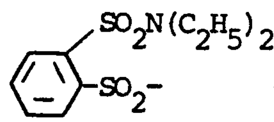 | $-OCHCH_2CH_3$ (mit $CH_3$ am CH) | 132 |
| 43 | O | 2 | H | $-NHOCH_3$ | H | $-O-CH_2-$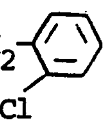 | 183 |
| 44 | O | 2 | H | $-N(C_2H_5)_2$ | H | $-NHCOCH_3$ | 164 |

Tabelle 1a

| Bsp.Nr. | 1:1 Addukt von Verbindungen der Formel (I) | Schmelzpunkt (°C) |
|---|---|---|
| 11a | $H_2SO_4$-Salz von Beispiel 11 | amorph |
| 22a | $CH_3SO_3H$-Salz von Beispiel 22 | 160 |
| 38a | $CH_3SO_3H$-Salz von Beispiel 38 | 179 |
| 17a | $H_2SO_4$-Salz von Beispiel 17 | 218 |

Herstellung von Ausgangsstoffen der Formel (II)

Beispiel (II-1)

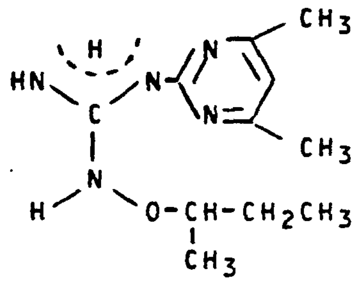

Eine Mischung aus 143g (0,97 Mol) 2-Cyanamino-4,6-dimethylpyrimidin, 94,3 g (1,06 Mol) O-sec.-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin anfallende Produkt wird durch Absaugen isoliert.

Man erhält 131g (57 % der Theorie) N'-(4,6-Dimethylpyrimidin-2-yl)-N''-(1-methylpropoxy)-guanidin vom Schmelzpunkt 52°C.

Beispiel (II-2)

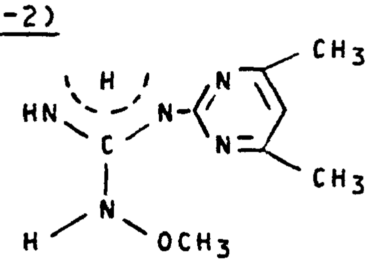

Eine Mischung aus 109 g (0,67 Mol) O-Methylhydroxylamin-Hydrochlorid, 99g (0,67 Mol) 2-Cyanamino-4,6-dimethylpyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8g (55 % der Theorie) N'-(4,6-Dimethylpyrimidin-2-yl)-N''-methoxy-guanidin vom Schmelzpunkt 134°C bis 136 °C.

Analog können die in der nachstehenden Tabelle aufgeführten Verbindungen der Formel (II) hergestellt werden:

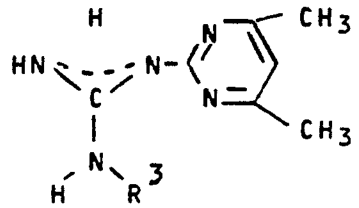

(II)

Tabelle 2:

| Bsp. Nr. | $R^3$ | Schmelzpunkt (°C) |
|---|---|---|
| II-3 | $-OCH_2CH(CH_3)_2$ | 78 |
| II-4 | $-OCH_2CH=CH_2$ | 103 |
| II-5 | $-OCH(CH_3)_2$ | 84 |
| II-6 | $-OCH_2CH_2-$ 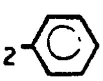 | $n_D^{24} = 1{,}5776$ |
| II-7 | $-O-C_8H_{17}-n$ | 58 |
| II-8 | $-O-CH_2-$ 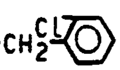 | 102-103 |
| II-9 | $-O-CH_2CH_2CH_2Cl$ | 137 |
| II-10 | -O- 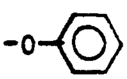 | 192 (Zers.) |
| II-11 | $-O-CH_2-COOCH_3$ | 148-149 |
| II-12 | $-O-CH_2-COOC_2H_5$ | 98-99 |
| II-13 | $-O-CH(CH_3)-COOCH_3$ | 147-148 |
| II-14 | $-O-CH_2-$ 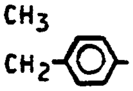 $-CH_3$ | 85-86 |

Tabelle 2 - Fortsetzung

| Beispiel Nr. | $R^3$ | Schmelzpunkt (°C) |
|---|---|---|
| II-15 | 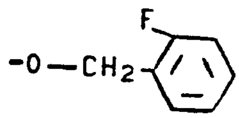 | 114- 116 |
| II-16 | 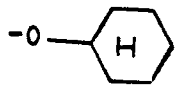 | |
| II-17 | 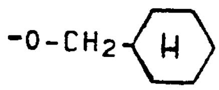 | |
| II-18 | $-O-CH_2CON(CH_3)_2$ | |
| II-19 | $-O-CH_2OCH_3$ | |
| II-20 | $-O-CH_2SCH_3$ | |
| II-21 | $-O-CH_2CF_3$ | |
| II-22 | 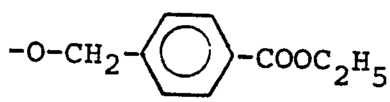 | 138 |
| II-23 | $-O-C_4H_9-n$ | Öl |
| II-24 | 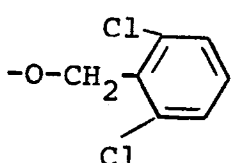 | 145 |
| II-25 | 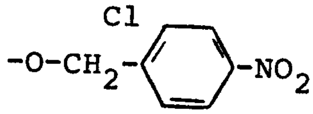 | 170-172 |
| II-26 | $-O-CH_2CH_2CH_3$ | 54 |
| II-27 | $-O-C_2H_5$ | 88 |
| II-28 | $-O-CH_2COOC_3H_7-i$ | 112 |
| II-29 | 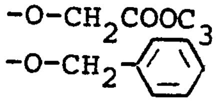 | 102 |

Herstellung von Ausgangsstoffen der Formel (III)

Beispiel (III-1)

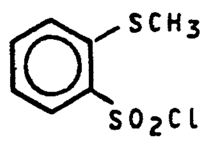

Eine Lösung von 38g (0,55 Mol) Natriumnitrit in 70 ml Wasser wird unter Rühren zu einer auf -5°C bis 0°C gekühlten Mischung aus 70g (0,5 Mol) 2-Methylthioanilin, 155 ml konzentrierter Salzsäure und 40 ml Essigsäure tropfenweise gegeben. Das Reaktionsgemisch wird 30 Minuten bei -5 bis 0°C gekühlt und dann tropfenweise zu einer Mischung aus 500 ml Essigsäure, 10g (0,1 Mol) Kupfer-(I)-chlorid und 200 g (3,1 Mol) Schwefeldioxid gegeben. Das Gemisch wird 3 Stunden bei 0°C gerührt und dann mit 1 l Toluol geschüttelt. Die organische Phase wird abgetrennt, mit Eiswasser gewaschen, getrocknet, filtriert und eingeengt.

Man erhält 80,5 g (72 % der Theorie) 2-Methylthio-benzolsulfonsäurechlorid in Form eines braungelben Oels, das ohne weitere Reinigung zu Umsetzungen verwendet wird.

Analog können die in der nachstehenden Tabelle 3 aufgeführten Verbindungen der Formel (III) hergestellt werden:

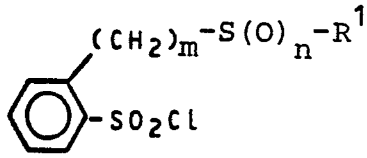

(III)

Tabelle 3:

| Bsp. Nr. | m | n | $R^1$ | Schmelzpunkt bzw. Siedepunkt/Druck |
|---|---|---|---|---|
| III-2 | 0 | 0 | $-CH(CH_3)_2$ | 90°C/1,33 mbar |
| III-3 | 0 | 2 | $-N(CH_3)_2$ | 103°C |
| III-4 | 1 | 1 | $-CH_3$ | |
| III-5 | 1 | 2 | $-CH_3$ | 120°C |
| III-6 | 0 | 2 | $-N(C_2H_5)_2$ | 73°C |
| III-7 | 0 | 2 | $-N(CH_3)(OCH_3)$ | |
| III-8 | 0 | 0 | $-C_2H_5$ | |
| III-9 | 1 | 1 | $-CH(CH_3)_2$ | |
| III-10 | 1 | 2 | $-CH(CH_3)_2$ | |
| III-11 | 0 | 2 | $-CH_3$ | 128°C |
| III-12 | 0 | 2 | $-C_2H_5$ | |
| III-13 | 0 | 2 | $-CH(CH_3)_2$ | |
| III-14 | O | O | $-CF_3$ | 41-43°C |
| III-15 | O | 2 | $-CF_3$ | |

Herstellung von Ausgangsstoffen der Formel (V)

Beispiel (V-1)

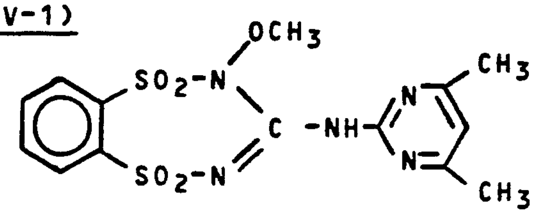

14g (0,05 Mol) Benzol-1,2-disulfonsäuredichlorid werden zu einer auf -20°C gekühlten Mischung aus 10g (0,05 Mol) N'-(4,6-Dimethyl-pyrimidin-2-yl)-N''-methoxy-guanidin, 12g (0,15 Mol) Pyridin und 100 ml Methylenchlorid unter Rühren gegeben. Das Reaktionsgemisch wird 3 Stunden bei -20°C und weitere 15 Stunden bei +20°C gerührt, dann mit eiskalter 5%iger Salzsäure und mit Wasser gewaschen, getrocknet, filtriert und eingeengt.
Das beim Verreiben des Rückstandes kristallin angefallene Produkt wird durch Absaugen isoliert.
Man erhält 11,5 g (58 % der Theorie) der Verbindung der oben angegebenen Strukturformel vom Schmelzpunkt 158°C.

Analog können die in der nachstehenden Tabelle 4 aufgeführten Verbindungen der Formel (V) hergestellt werden:

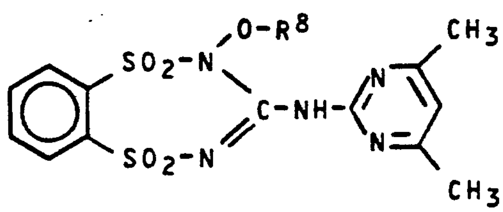

(V)

Tabelle 4:

| Bsp. Nr. | $R^B$ | Schmelzpunkt (°C) |
|---|---|---|
| V-2 | $-C_4H_9-n$ | 179 (Zers.) |
| V-3 | $-CH_2-$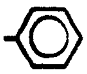 | 198 |
| V-4 | $-C_8H_{17}-n$ | 164 |
| V-5 | $-C_2H_5$ | 104 |
| V-6 | $-C_3H_7-n$ | 134 (Zers.) |
| V-7 | $-CH(CH_3)_2$ | amorph |
| V-8 | $-CH_2-CH=CH_2$ | 180 (Zers.) |
| V-9 | $-CH_2CH_2-$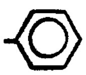 | |
| V-10 | 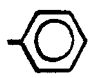 | |
| V-11 | $-CH_2-O-CH_3$ | |
| V-12 | $-CH_2-S-CH_3$ | |
| V-13 | $-CH_2-COOC_2H_5$ | 210 (Zers.) |

Beispiel :A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit: insbesondere die Verbindung aus Herstellungsbeispielen 1, 2, 5a, 17a.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit, insbesondere die Verbindung aus Herstellungsbeispielen 1, 2, 4, 5a, 17a.

Patentansprüche

1) Substituierte Arylsulfonylguanidine der allgemeinen Formel (I)

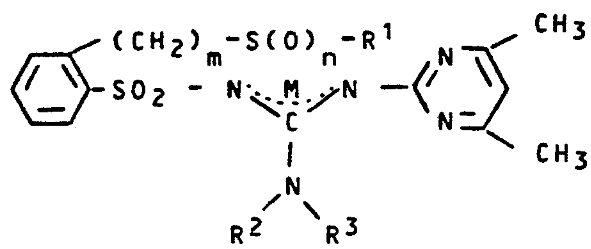

(I)

in welcher

m für Null oder 1 steht,

n für Null, 1 oder 2 steht,

M für Wasserstoff oder ein Metalläquivalent steht,

$R^1$ für $C_1$-$C_6$-Alkyl [welches gegebenenfalls durch Fluor und/oder Chlor substituiert ist], Amino, $C_1$-$C_4$-Alkylamino, $C_1$-$C_8$-Alkoxyamino, Di($C_1$-$C_4$-alkyl)-amino, $C_1$-$C_4$-Alkoxy-$C_1$-$C_4$-alkylamino, Benzyloxyamino oder Dimethylhydrazino steht,

$R^2$ für Wasserstoff, $C_1$-$C_6$-Alkyl [welches gegebenenfalls durch Fluor, Chlor, Brom, Cyano, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für den Rest

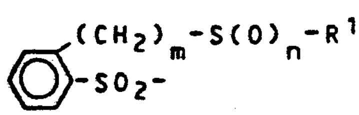

steht,

worin $R^1$ die oben angegebene Bedeutung hat
m für Null oder 1 steht und
n für Null, 1 oder 2 steht;
in welcher weiter

$R^3$ für Wasserstoff, $C_1$-$C_6$-Alkyl (welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-C6-Alkinyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht,oder

$R^2$ und $R^3$ gemeinsam für $C_4$-$C_6$-Alkandiyl stehen, welches gegebenenfalls durch eine Sauerstoff-Brücke oder durch eine Brücke >N-$R^4$ unterbrochen ist, wobei
$R^4$ für $C_1$-$C_4$-Alkyl, $C_1$-$C_4$-Alkyl-carbonyl oder Phenyl steht [welches gegebenenfalls durch Fluor, Chlor, Brom, Cyano, Nitro, $C_1$-$C_4$-Alkyl, Trifluormethyl oder $C_1$-$C_4$-Alkoxy substituiert ist];
in welcher weiter

$R^3$ für den Rest -O-$R^5$ steht, worin
$R^5$ für $C_1$-$C_{12}$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], $C_3$-$C_6$-Alkenyl, $C_1$-$C_4$-Alkoxy-carbonyl-$C_1$-$C_2$-

alkyl, Aminocarbonylmethyl, $C_1$-$C_4$-Alkylamino-carbonyl-methyl, Di-($C_1$-$C_4$-alkyl)-amino-carbonyl-methyl, Phenyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert ist] steht;
in welcher weiter

$R^3$ für den Rest $-N<^{R^6}_{R^7}$ steht, worin

$R^6$ für Wasserstoff oder $C_1$-$C_4$-Alkyl steht und

$R^7$ für Wasserstoff, $C_1$-$C_4$-Alkyl (gegebenenfalls durch Fluor, Chlor, Brom, Cyano, $C_1$-$C_4$-Alkoxy oder $C_1$-$C_4$-Alkoxycarbonyl substituiert ist], $C_3$-$C_6$-Cycloalkyl, Phenylethyl, Benzyl oder Phenyl [welches gegebenenfalls durch Fluor, Chlor, Brom, Nitro, Cyano, $C_1$-$C_4$-Alkyl, $C_1$-$C_4$-Alkoxy oder $C_1$-$C_4$-Alkoxy-carbonyl substituiert ist] steht,

sowie 1:1-Addukte von Verbindungen der Formel (I) mit starken Säuren.

2) Verfahren zur Herstellung von substituierten Arylsulfonylguanidinen der allgemeinen Formel (I) gemäß Anspruch 1, dadurch gekennzeichnet, daß man

(a) für den Fall, daß M für Wasserstoff steht und

$R^2$ für den Rest 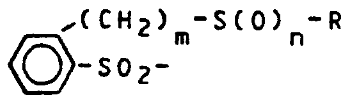 steht,

Guanidin-Derivate der Formel (II)

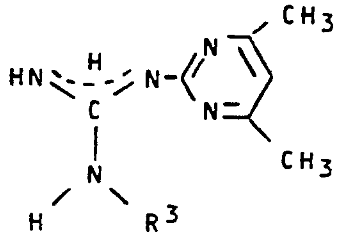 (II)

in welcher

$R^3$ die oben angegebene Bedeutung hat,

mit wenigstens zwei Moläquivalenten von substituierten Arensulfonsäurechloriden der Formel (III)

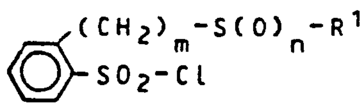 (III)

in welcher

$R^1$ sowie m und n die oben angegebene Bedeutung haben,

in Gegenwart von Säureakzeptoren und gegebenenfalls in Gegenwart von Verdünnungsmitteln umsetzt;

oder daß man

(b) für den Fall, daß M für Wasserstoff steht und $R^2$ für Wasserstoff, $C_1$-$C_6$-Alkyl [welches gegebenenfalls wie oben angegeben substituiert ist], $C_3$-$C_6$-Cycloalkyl, $C_3$-$C_6$-Alkenyl oder Benzyl [ welches gegebenenfalls wie oben angegeben substituiert ist] steht oder zusammen mit $R^3$ die oben angegebene Bedeutung hat,

die nach dem unter (a) angegebenen Verfahren erhältlichen substituierten Arylsulfonylguanidine der Formel (ID)

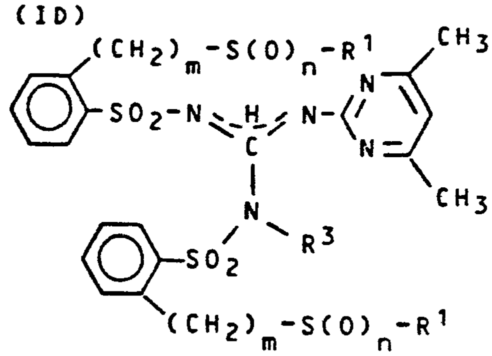

(ID)

in welcher

$R^1$, $R^3$, m und n die oben angegebene Bedeutung haben,

mit Aminoverbindungen der Formel (IV)

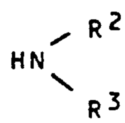

(IV)

in welcher

$R^2$ die vorausgehend unter (b) angegebene Bedeutung hat und

$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder daß man

(c) für den Fall, daß m für Null steht, n für 2 steht, $R^1$ für $C_1$-$C_8$-Alkoxyamino oder Benzyloxyamino steht und $R^2$ für Wasserstoff, $C_1$-$C_6$-Alkyl [welches gegebenenfalls wie oben angegeben substituiert ist], $C_3$-$C_6$-Cycloalkyl, $C_3$-$C_6$-Alkenyl oder Benzyl [welches gegebenenfalls wie oben angegeben substituiert ist] steht oder zusammen mit $R^3$ die oben angegebene Bedeutung hat,

Verbindungen der Formel (V)

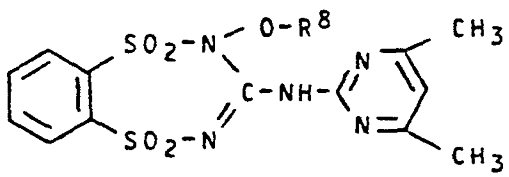

(V)

in welcher

$R^8$ für $C_1$-$C_8$-Alkyl oder Benzyl steht,

mit Aminoverbindungen der Formel (VI)

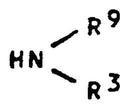

(VI)

in welcher

$R^9$ die oben unter (c) für $R^2$ angegebene Bedeutung hat und

$R^3$ die oben angegebene Bedeutung hat,

gegebenenfalls in Gegenwart von Verdünnungsmitteln umsetzt; oder daß man

(d) für den Fall, daß M für ein Metalläquivalent steht, die nach den oben unter (a), (b) und (c) angegebenen Verfahren erhältlichen Verbindungen der Formel (I), in welcher M für Wasserstoff steht und $R^1$, $R^2$, $R^3$, m und n die oben angegebenen Bedeutungen haben,

mit Metall-hydroxiden, -hydriden oder -alkanolaten oder mit metallorganischen Verbindungen gegebenenfalls in Gegenwart von Verdünnungsmitteln umsetzt;

oder daß man

(e) für dem Fall, daß 1:1-Addukte von Verbindungen der Formel (I) mit starken Säuren herzustellen sind,

Verbindungen der Formel (I) in welcher M für Wasserstoff steht und $R^1$, $R^2$, $R^3$, m und n die oben angegebenen Bedeutungen haben,

mit starken Säuren gegebenenfalls in Gegenwart von Verdünnungsmitteln umsetzt.

3.) Herbizide Mittel, gekennzeichnet durch einen Gehalt an mindestens einem substituierten Arylsulfonylguanidin der allgemeinen Formel (I) gemäß Anspruch 1.

4.) Verwendung von substituierten Arylsulfonylguanidinen der allgemeinen Formel (I) gemäß Anspruch 1 zur Bekämpfung von unerwünschtem Pflanzenwachstum.

5.) Verfahren zur Herstellung von herbiziden Mitteln, dadurch gekennzeichnet, daß man substituierte Arylsulfonylguanidine der allgemeinen Formel (I) gemäß Anspruch 1 mit Streckmitteln und/oder oberflächenaktiven Mitteln vermischt.

Europäisches Patentamt

# 0 173 319

## EUROPÄISCHER RECHERCHENBERICHT

EP 85110831.6

| Kategorie | EINSCHLÄGIGE DOKUMENTE – Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y | EP - A1 - 0 117 014 (E.I. DU PONT DE NEMOURS)<br>* Zusammenfassung; Formel II; Tabellen 23, 25 *<br>-- | 1,3-5 | C 07 D 239/42<br>A 01 N 47/44 |
| P,Y | EP - A1 - 0 148 498 (NIHON TOKUSHU NOYAKU SEIZO)<br>* Zusammenfassung * | 1,3-5 | |
| P,A | * Patentanspruch 5 *<br>-- | 2 | |
| D,A | DD - A - 71 016 (KOCHMANN)<br>* Spalte 1, Zeilen 21-29 *<br>---- | 1,3 | |

RECHERCHIERTE SACHGEBIETE (Int. Cl. 4)

C 07 D 239/00
A 01 N 47/00

Der vorliegende Recherchenbericht wurde für alle Patentansprüche erstellt.

| Recherchenort | Abschlußdatum der Recherche | Prüfer |
|---|---|---|
| WIEN | 22-11-1985 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82